(19) Europäisches Patentamt
European Patent Office
Office européen des brevets

(11) EP 1 237 703 B1

(12) # EUROPÄISCHE PATENTSCHRIFT

(45) Veröffentlichungstag und Bekanntmachung des Hinweises auf die Patenterteilung:
**13.05.2009 Patentblatt 2009/20**

(21) Anmeldenummer: **00993390.4**

(22) Anmeldetag: **15.12.2000**

(51) Int Cl.:
***B29C 45/54** (2006.01)* ***A61J 3/06** (2006.01)*
***A61K 9/20** (2006.01)*

(86) Internationale Anmeldenummer:
**PCT/EP2000/012797**

(87) Internationale Veröffentlichungsnummer:
**WO 2001/043943 (21.06.2001 Gazette 2001/25)**

(54) **VERFAHREN ZUM HERSTELLEN VON FESTEN WIRKSTOFFHALTIGEN FORMEN**

METHOD FOR PRODUCING SOLID SHAPES CONTAINING AN ACTIVE INGREDIENT

PROCEDE DE PRODUCTION DE CORPS MOULES SOLIDES CONTENANT UN PRINCIPE ACTIF

(84) Benannte Vertragsstaaten:
**AT BE CH CY DE DK ES FI FR GB GR IE IT LI LU MC NL PT SE TR**

(30) Priorität: **15.12.1999 DE 19960494**

(43) Veröffentlichungstag der Anmeldung:
**11.09.2002 Patentblatt 2002/37**

(73) Patentinhaber: **Abbott GmbH & Co. KG**
**65205 Wiesbaden (DE)**

(72) Erfinder:
- **KRULL, Harald**
  **67071 Ludwigshafen (DE)**
- **ROSENBERG, Jörg**
  **67158 Ellerstadt (DE)**
- **BREITENBACH, Jörg**
  **68199 Mannheim (DE)**
- **HOFMANN, Jürgen**
  **67069 Ludwigshafen (DE)**
- **KLENZ, Rainer**
  **67454 Hassloch (DE)**
- **BÜHRLE, Hans**
  **67117 Limburgerhof (DE)**

(74) Vertreter: **Kinzebach, Werner et al**
**Reitstötter, Kinzebach & Partner**
**Patentanwälte**
**Sternwartstrasse 4**
**81679 München (DE)**

(56) Entgegenhaltungen:

| | |
|---|---|
| **EP-A- 0 337 256** | **WO-A-95/11122** |
| **WO-A-98/41381** | **CH-A- 358 585** |
| **DE-A- 2 335 973** | **DE-A- 19 606 490** |
| **DE-A- 19 903 614** | **DE-B- 1 142 229** |
| **GB-A- 580 097** | **US-A- 5 885 691** |

- **PATENT ABSTRACTS OF JAPAN vol. 8, no. 30 (C-209), 8. Februar 1984 (1984-02-08) & JP 58 192817 A (NIPPON SODA KK), 10. November 1983 (1983-11-10)**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 237 703 B1

Printed by Jouve, 75001 PARIS (FR)

## Beschreibung

Die vorliegende Erfindung betrifft ein Verfahren zum Herstellen von festen wirkstoffhaltigen Formen aus einer wirkstoffhaltigen Formulierung, die mindestens ein polymeres Bindemittel enthält.

Herkömmlicherweise werden die oben beschriebenen festen pharmazeutischen Formen dadurch hergestellt, daß zunächst eine wirkstoffhaltige Formulierung mittels eines Schmelzextrusionsverfahrens bereitgestellt wird und danach die Formgebung erfolgt. Bei der Formgebung werden die festen Formen aus der mit der Extrusion erhaltenen wirkstoffhaltigen Schmelze beispielsweise mittels gegenläufig rotierenden Kalanderwalzen gebildet. Eine derartige Vorrichtung ist in der WO 97/15268 beschrieben.

Des weiteren werden in der Kunststofftechnik Formen durch Spritzguß hergestellt. Diese aus der Kunststoffverarbeitung bekannten Vorrichtungen und Verfahren lassen sich jedoch nicht ohne weiteres zur Herstellung von wirkstoffhaltigen Formen einsetzen. Im Gegensatz zum kontinuierlich ablaufenden Verfahren beim Einsatz der Kalandrierung muß nämlich beim Einsatz des Spritzgußverfahrens ein Teil des Prozeßschrittes diskontinuierlich erfolgen, da die Schmelze unter hohem Druck in ausgeformte Hohlkörper gedrückt wird und dort erkalten muß, bevor die Kavitäten des Formwerkzeugs erneut mit neuer Schmelze beschickt werden können. Dies wirkt sich nachteilig auf die Herstellung von wirkstoffhaltigen Formen aus, da die Verweilzeiten der wirkstoffhaltigen Schmelze bei den zur Schmelzerzeugung notwendigen hohen Temperaturen sehr viel länger sind, als im Fall des Kalandrierverfahrens, bei dem die Schmelze kontinuierlich ohne Unterbrechung in den Formkalander gefördert wird. Die langen Verweilzeiten der Schmelze im Extruder bis zur Abkühlung in den Formkavitäten führen im Fall empfindlicher Wirkstoffe zur Bildung erheblicher Anteile von Zersetzungsprodukten, die bei wirkstoffhaltigen Formen nicht auftreten dürfen.

Die WO 95/11122 A1 beschreibt ein Verfahren zur Herstellung von Kunststoffprodukten, bei dem die physikalischen Eigenschaften des Produkts beeinflusst werden können. Bei einem Ausführungsbeispiel dieser Druckschrift ist ein Extruder vorgesehen, mit dem wechselweise zwei Akkumulatoren beschickt werden können. Mittels der Akkumulatoren wird bei der Kunststoffmasse eine Scherspannung und ein Spannungstensor erzeugt und eine Druckkraft ausgeübt. Ferner wird die Kunststoffmasse mittels der Akkumulatoren in eine Form eingespritzt.

Aus der WO 98/41381 A1 ist ein weiteres Verfahren zur Herstellung von Kunststoffprodukten bekannt. Bei dem Verfahren soll der Gehalt des bei der Kunststoffherstellung anfallenden Nebenprodukts Acetalaldehyd verringert werden. Bei dem Verfahren wird die Kunststoffmasse einem Extruder zugeführt. Am Ende des Extruders befindet sich eine Öffnung, bei welcher ein Mittel zum Verringern des Acetalaldehydgehalts zugegeben wird. Von dem Extruder wird die Kunststoffmasse mittels einer Pumpe wechselseitig zwei Spritzeinheiten zuführt, bei denen sie in Formen gespritzt wird.

In der EP 0 337 256 A2 ist ein Verfahren zur Herstellung pharmazeutischer Mischungen durch kontinuierliches Dosieren der Komponenten beschrieben. Die Formgebung erfolgt bei diesem Verfahren dadurch, dass man die Mischung extrudiert und den noch verformbaren Strang zwischen zwei gegenläufig angetriebenen Walzen verpresst.

In der EP 240 904 B1 wird bei einem Verfahren zur Herstellung von festen wirkstoffhaltigen Formen auf die Spritzgußtechnik hingewiesen, jedoch werden dort keine näheren Angaben dazu gemacht, wie eine Vorrichtung oder ein Verfahren im Einzelnen ausgebildet sein könnten, mit denen die vorstehenden Nachteile vermieden werden können.

Es ist die Aufgabe der vorliegenden Erfindung, ein Verfahren zum Herstellen von festen wirkstoffhaltigen Formen anzugeben, bei denen die Spritzgußtechnik benutzt wird, jedoch die damit verbundenen Nachteile vermieden werden.

Diese Aufgabe wird erfindungsgemäß durch ein Verfahren gemäß Anspruch 1 gelöst, wobei sich vorteilhafte Ausgestaltungen aus den unteransprüchen ergeben.

Eine Vorrichtung zur Durchführung des Verfahrens ist durch zumindest zwei getrennt voneinander vorgesehene, jeweils mit dem Extruder verbundene Spritzeinheiten gekennzeichnet, über die die Formulierung in zumindest ein Formwerkzeug einspritzbar ist.

Das Bereitstellen von zumindest zwei getrennt voneinander vorgesehenen Spritzeinheiten verhindert vorteilhafterweise, daß die wirkstoffhaltige Formulierung zu lange im Extruder verbleibt, in dem zur Plastifizierung der Formulierung so hohe Temperaturen herrschen, daß bei längeren Verweilzeiten der Formulierung in dem Extruder Zersetzungsprodukte gebildet werden. Die plastifizierte wirkstoffhaltige Formulierung kann bei der Vorrichtung sehr viel schneller in eine Spritzeinheiten gelangen, da immer die Spritzgießeinheit beschickt werden kann, die gerade keine Formulierung in ein Formwerkzeug einspritzt. Die Vorrichtung verbindet somit den kontinuierlich betreibbaren Extruder mit mehreren diskontinuierlich arbeitenden Spritzeinheiten, wobei die sich aus den Diskontinuitäten ergebenden Nachteile vermieden werden. Hierdurch wird des weiteren die Herstellungsrate der wirkstoffhaltigen Formen erhöht. Insbesondere beim Spritzgießen von wirkstoffhaltigen Formen mit kurzer Abkühlzeit und hohen Schußgewichten bzw. hohen Stückzahlen, wie z.B. bei Tabletten, kann die Effizienz, mit der die wirkstoffhaltigen Formen hergestellt werden, erheblich gesteigert werden. Mit der Vorrichtung können sehr hohe Produktionsraten von bis zu 400.000 wirkstoffhaltigen Formen pro Stunde erreicht werden. Übliche Produktionsraten der Vorrichtung liegen über 50.000 Stück pro Stunde und vorteilhafterweise zwischen 100.000 und 300.000 Stück pro Stunde. Die Massen der wirkstoffhaltigen Formen liegen dabei üblicherweise zwischen 50 und 2000 mg, meist jedoch zwischen 150 und 1300 mg.

Des weiteren wird bei einem kontinuierlich betreibbaren Extruder die schmelzequalität im Vergleich zu einem nur diskontinuierlich betreibbaren Extruder erhöht, da die Plastifizierbedingungen, wie die Schneckendrehzahl des Extruders, die Temperatur, die plastifizierende Schneckenlänge und der Durchsatz, konstant bleiben.

In einer vorteilhaften Ausgestaltung der vorliegenden Erfindung kann zumindest eine Spritzeinheit mit mehreren, parallel geschalteten Formwerkzeugen in Verbindung stehen, in die die Formulierung gleichzeitig einspritzbar ist. Des weiteren kann zumindest eine Spritzeinheit mit mehreren, einzeln an die Spritzeinheit ankoppelbaren Formwerkzeugen in Verbindung stehen, in die die Formulierung nacheinander einspritzbar ist. Ferner können mehrere Spritzeinheiten wechselweise an ein Formwerkzeug ankoppelbar sein. Ist die Schmelzeplastifizierung der geschwindigkeitsbestimmende Schritt kann durch diese Ausgestaltung vorteilhafterweise die Herstellungsrate der wirkstoffhaltigen Form erhöht werden.

Ist der spritzgießvorgang der geschwindigkeitsbestimmende Schritt, z.B. bei der Herstellung von kleinen wirkstoffhaltigen Formen mit langen Abkühlzeiten kann das Vorsehen von mehreren Formwerkzeugen pro Spritzeinheit die Herstellrate erhöhen.

In einer weiteren Ausgestaltung ist das oder sind die Formwerkzeuge als Etagenwerkzeuge mit jeweils mehreren Einzelwerkzeugen ausgebildet. Auch bei dieser Ausgestaltung kann vorteilhafterweise die Herstellungsrate erhöht werden, wenn kleine wirkstoffhaltige Formen mit verhältnismäßig langen Abkühlzeiten hergestellt werden sollen.

In einer weiteren erfindungsgemäßen Ausgestaltung entspricht jeweils das Volumen eines Zylinder einer Spritzeinheit dem Volumen des Formwerkzeugs oder der Formwerkzeuge, in die die Formulierung von der jeweiligen Spritzeinheit einspritzbar ist.

Da bei dieser Ausgestaltung bei jedem Einspritzvorgang die gesamte in der Spritzeinheit befindliche Formulierung ausgestoßen wird, wird vorteilhafterweise vermieden, daß eine heiße wirkstoffhaltige Formulierung zu lange in einer Spritzeinheit verbleibt, so daß sich entweder der Wirkstoff oder das polymere Bindemittel zersetzen kann. Des weiteren wird hierdurch die Zeitdauer zwischen dem Füllen des Zylinders und dem Einspritzen in das Formwerkzeug so kurz wie möglich gehalten.

In einer weiteren Ausgestaltung ist zwischen dem Extruder und den Spritzeinheiten eine Speichereinheit vorgesehen zum Zwischenspeichern der von dem Extruder kontinuierlich erzeugten Formulierung und zum Weiterleiten der Formulierung an die diskontinuierlich betriebenen Spritzeinheiten. Zwar wird durch diese Ausgestaltung die Zeit vom Extrudieren bis zum Einspritzvorgang etwas verlängert, jedoch kann auf diese Weise in jedem Fall der kontinuierliche Betrieb des Extruders sichergestellt werden. Durch die kontinuierliche Fahrweise werden die Eigenschaften der plastifizierten Formulierung verbessert.

Durch die Verwendung einer Speichereinheit ist es außerdem möglich, den kontinuierlich arbeitenden Extruder mit nur einer diskontinuierlichen Spritzeinheit zu verbinden, da der Zwischenspeicher den Extruder und die Spritzeinheit entkoppelt. Durch die Entkopplung ist eine kontinuierliche Plastifizierung im Extruder möglich.

In einer weiteren erfindungsgemäßen Ausgestaltung ist zwischen dem Extruder und den Spritzeinheiten ein Verteilerventil vorgesehen, dem die von dem Extruder plastifizierte Formulierung zuführbar ist und über das die zugeführte Formulierung auf die Spritzeinheiten verteilbar ist. Durch das Vorsehen des Verteilerventils wird die Entkopplung von einem kontinuierlich betriebenen Extruder von den diskontinuierlich betriebenen Spritzeinheiten gewährleistet.

In einer weiteren Ausführungsform der Vorrichtung weist diese mehrere Extruder zum kontinuierlichen Plastifizieren unterschiedlicher Massen auf, wobei zumindest eine Masse die wirkstoffhaltige Formulierung ist und wobei die mehreren Extruder jeweils mit einer oder mehreren Spritzeinheiten in Verbindung stehen und ein Formwerkzeug mit zumindest zwei Spritzeinheiten in Verbindung steht, denen Massen von verschiedenen Extrudern zuführbar sind. Vorteilhafterweise ist in diesem Fall das Formwerkzeug als Negativform einer herzustellenden wirkstoffhaltigen Form ausgebildet, wobei die wirkstoffhaltige Form im Wesentlichen die Form einer halben Kugel mit Hohlraum oder eines halben Hohlzylinders besitzt. Ferner können in diesem Fall dem Formwerkzeug die Massen der Spritzeinheiten so zuführbar sein, dass bei der hergestellten Form der Wirkstoff nur über die Oberfläche der Innenwandung der Hohlkugel bzw. des Hohlzylinders abgebbar ist.

Durch diese Ausführungsform ist ein Mehrkomponentenspritzguß möglich. Dabei kann z.B. eine Form aus zwei oder mehr verschiedenen wirkstoffhaltigen Formulierungen, die einen oder mehrere Wirkstoff(e) enthalten, gebildet werden, wobei nacheinander Schichten mit verschiedenen Formulierungen gebildet werden können. Auf diese Weise ist es z.B. möglich, wirkstoffhaltige Formen mit einem mehrphasigen Freisetzungsprofil oder einem Freisetzungsprofil 0. Ordnung herzustellen, wobei letzteres von besonderem Interesse ist. Während bei konventionellen wirkstoffhaltigen Formen wie z.B Tabletten aufgrund der oberflächenreduzierung mit zunehmender Auflösung der Form die im wesentlichen von der Oberfläche der Form abhängige Wirkstoffabgabe pro Zeiteinheit abnimmt (Wirkstofffreigabe nicht 0. Ordnung), können über den Mehrkomponenten-Spritzguss Formen hergestellt werden, die aufgrund der Konstanz der wirkstoffabgebenden Oberfläche eine konstante Wirkstofffreigabe pro Zeiteinheit besitzen (Wirkstofffreigabe 0. Ordnung). Formen, die eine Wirkstoff-Freigabe 0. Ordnung aufweisen, sind bereits von W.D. Rhine et al., in "Controlled Release of Bioactive Materials", Academic Press, 1980, ISBN 0-12-074450-3 beschrieben worden.

Mit dem Mehrkomponenten-Spritzguss ist weiterhin ein direktes Beschichten der wirkstoffhaltigen Formen mit verschiedenen Formulierungen möglich, wobei diese Formulierungen auch wirkstoffe enthalten können.

In einer weiteren Ausführungsform der Vorrichtung ist der oder sind die Extruder Doppelschneckenextruder. Als Doppelschneckenextruder werden hier Extruder mit zwei nebeneinander liegenden Schnecken bezeichnet, die in entgegengesetzter oder gleicher Drehrichtung rotieren. Vorteilhaft an der Verwendung von Doppelschneckenextrudern im Vergleich zu Einschneckenextrudern ist, daß diese sich besonders gut zur Verarbeitung der wirkstoffhaltigen Ausgangsstoffe eignen. Diese Ausgangsstoffe oder Stoffgemische für wirkstoffhaltige Formen sind nahezu ausschließlich pulverförmig, was zu einer erheblich niedrigeren Schüttdichte im Vergleich zu derjenigen bei Kunststoffgranulaten führt. Dies führt zu Problemen bei der Befüllung, falls in der Kunststoffverarbeitung verwendete Extruder zur Herstellung der wirkstoffhaltigen Formen verwendet werden. Doppelschneckenextruder besitzen beim Einziehen pulverförmiger Stoffgemische mit niedrigen Dichten wesentlich bessere Eigenschaften als Einschneckenextruder. Dies führt zu einem erheblich besseren Schmelzefluß in dem Extruder. Des weiteren ist es bei der Verwendung von Einschneckenextrudern erforderlich, die aufzuschmelzende, wirkstoffhaltige Formulierung zuvor zu granulieren, um die Schüttdichte zu erhöhen. Dies ist bei der Verwendung von Doppelschneckenextrudern vorteilhafterweise nicht mehr erforderlich.

Als Spritzgießeinheiten werden vorteilhafterweise zumindest teilweise Kolbeneinspritzeinheiten verwendet. Bei derartigen Kolbeneinspritzeinheiten kann besser als bei Schneckeneinspritzeinheiten gewährleistet werden, daß nach dem Einspritzvorgang keine wirkstoffhaltige Formulierung im Kolben verbleibt und sich dabei der Wirkstoff oder das polymere Bindemittel zersetzt.

Erfindungsmäß werden die Spritzeinheiten und die Formwerkzeuge über temperatursteuerbare oder temperaturegelbare Heißkanalsysteme verbunden. Der Einsatz von Heißkanalsystemen besitzt den Vorteil, daß die plastifizierte Masse nur in den eigentlichen Formkavitäten der Spritzgußform erstarrt, nicht aber in den Schmelzeverteilerkanälen. Auf diese Weise kann besonders beim Spritzen kleiner Formen die Ausbeute erheblich vergrößert werden, da der sonst anfallenden Anguß entfällt und somit die Herstellung nahezu ausschussfrei ist. Ein weiterer Vorteil ist eine Verkürzung der Zykluszeit und somit eine Steigerung der Produktionsraten, da die meist zykluszeitbestimmende Abkühlung der Angüsse entfällt.

Für eine wirtschaftliche Herstellung wirkstoffhaltiger Formen mit Hilfe des Spritzgusses spielt die Heißkanaltechnik eine wichtige Rolle, da durch deren Verwendung der Ausschuss an den meist teuren Wirkstoffen auf ein Minimum reduziert und aufgrund des fehlenden Angusses die Zykluszeit merkbar verkürzt werden kann, was eine Steigerung der Produktionsraten mit sich bringt.

Die Heißkanalsysteme bestehen aus einem Verteilerbereich und Heißkanaldüsen, die Einspritzkanäle zum Einspritzen der Massen in die Kavitäten des Formwerkzeugs enthalten. Für die Herstellung wirkstoffhaltiger Formen werden an den Heißkanalsystemen bevorzugt Temperaturen zwischen 50 und 250°C, besonders bevorzugt zwischen 100 und 200°C eingestellt.

Die Temperatursteuerung oder -regelung des Heißkanals erfolgt mittels eines Fluids oder einer Elektroheizung, wobei im letzteren Fall eine Gegenkühlung vorgesehen wird.

Unabhängig von der Verwendung eines Heißkanalsystems können des weiteren auch die in der Regel elektrobeheizten Spritzeinheiten, die Auslaßöffnung des Extruders und/oder das Verteilerventil über eine Fluidtemperierung temperatursteuerbar oder -regelbar sein.

Bei Vorrichtungen zum Herstellen von festen wirkstoffhaltigen Formen aus einer wirkstoffhaltigen Formulierung, in der die Spritzgußtechnik Anwendung findet, tritt das Problem auf, daß es beim Plastifizieren der Formulierung und beim Einspritzen dieser in ein Formwerkzeug zu Temperaturerhöhungen kommen kann, die die wirkstoffhaltige Formulierung schädigen. Die wirkstoffhaltige Formulierung wird im Extruder durch den Plastifiziervorgang erhitzt und über die Spritzeinheit in das Formwerkzeug eingespritzt. Dabei wird die Temperatur der Formulierung durch Kompression und durch den Einströmvorgang aufgrund innerer Reibung erhöht. Diesen nachteiligen Temperaturerhöhungen können durch das Vorsehen einer Temperatursteuerung und -regelung gemäß der erfindungsgemäßen Vorrichtung entgegengewirkt werden.

Wird eine elektrische Temperatursteuerung und -regelung verwendet, kann eine Überhitzung der Formulierung aufgrund von Kompression bzw. innere Reibung während des Einspritzvorganges durch die Installation einer Gegenkühlung verhindert werden.

Besonders wirkungsvoll ist die Temperatursteuerung und -regelung durch eine Fluidtemperierung, da sowohl die Überhitzung der Formulierung durch Kompression und innere Reibung als auch die bei elektrischen Heizsystemen auftretende lokale Überhitzung der Formulierung aufgrund des im Gegensatz zu elektrischen Heizsystemen gleichmäßigeren Wärmeübergangs zwischen Fluid und Formulierung verhindert werden können.

Die vorstehend beschriebene Vorrichtung kann insbesondere zur Erzeugung von wirkstoffhaltigen Formen mit einer Wirkstoff-Freisetzung 0. Ordnung verwendet werden. Des weiteren kann sie zur Erzeugung von wirkstoffhaltigen Formen mit einem mehrphasigen Freisetzungsprofil verwendet werden.

Bei dem erfindungsgemäßen Verfahren zum Herstellen von festen wirkstoffhaltigen Formen aus einer wirkstoffhaltigen Formulierung, die mindestens ein polymeres Bindemittel enthält, wird die wirkstoffhaltige Formulierung kontinuierlich plastifiziert, die plastifizierte Formulierung einer Spritzeinheit zugeführt und von dort in ein Formwerkzeug eingespritzt. Des weiteren wird die plastifizierte Formulierung einer weiteren Spritzeinheit zugeführt und von dort in ein Formwerkzeug eingespritzt. Die verschiedenen Spritzeinheiten können die Formulierung nacheinander in dasselbe Formwerkzeug oder in verschiedene Formwerkzeuge einspritzen. Vorteilhafterweise entspricht das einer Spritzeinheit zugeführte Formulierungsvolumen dem in das Formwerkzeug eingespritzte Volumen.

In einer Ausbildung des erfindungsgemäßen Verfahrens wird die plastifizierte Formulierung zwischengespeichert und danach einer Spritzeinheit zugeführt.

In einer weiteren Ausgestaltung können in ein Formwerkzeug unterschiedliche Massen eingespritzt werden, wobei zumindest eine Masse eine wirkstoffhaltige Formulierung enthält. Ferner können vor dem Einspritzen zwei Folien in das Formwerkzeug eingelegt werden, zwischen die die Formulierung eingespritzt wird.

Die wirkstoffhaltige Formulierung kann vor dem Einspritzen, beispielsweise beim Plastifizieren, ferner gemischt und/oder homogenisiert werden. Des weiteren kann die Temperatur der Formulierung beim Einspritzen, beim Plastifizieren, beim Zuführen und/oder beim Zwischenspeichern gesteuert oder geregelt werden.

Erfindungsgemäß wird für die Herstellung der wirkstoffhaltigen Form ein geeignetes polymeres Bindemittel verwendet. Das Bindemittel kann in Wasser quellbar oder wasserlöslich sein.

Erfindungsgemäß brauchbare Bindemittel sind Polyvinyllactame, insbesondere Polyvinylpyrrolidon (PVP), Copolymere von vinyllactamen, wie N-Vinylpyrrolidon, N-Vinylpiperidon und N-Vinyl-ε-caprolactam, aber insbesondere N-Vinylpyrrolidon, mit (Meth)acrylsäure, (Meth)acrylsäureestern, Vinylestern, insbesondere Vinylacetat, Copolymerisate von Vinylacetat und Crotonsäure, teilverseiftes Polyvinylacetat, Polyvinylalkohol, Polyhydroxyalkylacrylate, Polyhydroxyalkylmethacrylate, Polyacrylate und Polymethacrylate, Copolymerisate von Dimethylaminoethylacrylaten und Methacrylestern (z.B. Eudragit-Typen), Polyalkylenglykole, wie Polypropylenglykole und Polyethylenglykole (z.B. Polyethylenglykol 6000), Copolymerisate von Methylmethacrylat und Acrylsäure, Celluloseester, Celluloseether, insbesondere Methylcellulose und Ethylcellulose, Hydroxyalkylcellulosen, insbesondere Hydroxypropylcellulose oder Hydroxypropylmethylcellulose, Hydroxyalkyl-Alkylcellulosen, insbesondere Hydroxypropyl-Ethylcellulose, Cellulosephthalate, insbesondere Celluloseacetatphthalat und Hydroxypropylmethylcellulosephthalat, und Mannane, insbesondere Galactomannane.

Brauchbar sind auch Gelatine und bioabbaubare Polymere, wie Polyhydroxyalkanoate, z.B. Polyhydroxybuttersäure, Polymilchsäure, Polyaminosäuren, z.B. Polylysin, Polyasparagin, Polydioxane und Polypeptide.

Bevorzugte polymere Bindemittel sind Polyvinylpyrrolidon, Copolymerisate von N-Vinyllactamen, insbesondere N-Vinylpyrrolidon, und Vinylestern, Copolymerisate von N-Vinyllactamen, insbesondere N-Vinylpyrrolidon, mit (Meth) acrylsäureestern, Polyhydroxyalkylacrylate, Polyhydroxyalkylmethacrylate, Polyacrylate, Polymethacrylate, Alkylcellulosen und Hydroxyalkylcellulosen.

Vorteilhaft am erfindungsgemäßen Verfahren ist, dass es sich für Bindemittel sehr unterschiedlicher Viskosität eignet, beispielsweise für Bindemittel mit K-Werten (nach H. Fikentscher, Cellulose-Chemie 13 (1932), S. 58-64 und 71-74) zwischen 10 und 100, insbesondere zwischen 20 und 100. Insbesondere kommen die Vorteile dieses Verfahrens zum Tragen bei Bindemitteln, die einen K-Wert von > 45 und bevorzugt von > 50 aufweisen.

Die Formulierung kann neben dem polymeren Bindemittel übliche Zusätze enthalten, wie Weichmacher, galenische Hilfsstoffe, wie Schmiermittel, Trennmittel, Fließmittel, Farbstoffe, Stabilisatoren etc.

Beispiele für derartige Weichmacher sind:

langkettige Alkohole, Ethylenglykol, Propylenglykol, Glycerin, Trimethylolpropan, Triethylenglykol, Butandiole, Pentanole, wie Pentaerythrit, Hexanole, Polyethylenglykole, Polypropylenglykole, Polyethylen-propylenglykole, Silicone, aromatische Carbonsäureester (z.B. Dialkylphthalate, Trimellithsäureester, Benzoesäureester, Terephthalsäureester) oder aliphatische Dicarbonsäureester (z.B. Dialkyladipate, Sebacinsäureester, Azelainsäureester, Zitronen- und Weinsäureester), Fettsäureester, wie Glycerinmono-, Glycerindi- oder Glycerintriacetat oder Natriumdiethylsulfosuccinat. Die Konzentration an Weichmacher beträgt im Allgemeinen 0,5 bis 15, vorzugsweise 0,5 bis 5 Gew.-%, bezogen auf das Gesamtgewicht des Gemisches.

Übliche galenische Hilfsstoffe, deren Gesamtmenge bis zu 100 Gew.-% bezogen auf das Polymerisat, betragen kann, sind z.B. Streckmittel bzw. Füllstoffe, wie Silikate oder Kieselerde, Magnesiumoxid, Aluminiumoxid, Titanoxid, Methylcellulose, Natrium-Carboxymethylcellulose, Talkum, Saccharose, Lactose, Getreide- oder Maisstärke, Kartoffelmehl, Polyvinylalkohol, insbesondere in einer Konzentration von 0,02 bis 50, vorzugsweise 0,20 bis 20 Gew.-%, bezogen auf das Gesamtgewicht des Gemisches;

Schmiermittel und Trennmittel wie Magnesium-, Aluminium- und Calciumstearat, Talkum und Silicone, sowie tierische oder pflanzliche Fette, insbesondere in hydrierter Form und solche, die bei Raumtemperatur fest sind. Diese Fette haben vorzugsweise einen Schmelzpunkt von 50°C oder höher. Bevorzugt sind Triglyceride der $C_{12}$-, $C_{14}$-, $C_{16}$- und $C_{18}$-Pettsäuren. Auch Wachse, wie Carnaubawachs, sind brauchbar. Diese Fette und Wachse können vorteilhaft alleine oder zusammen mit Mono- und/oder Diglyceriden oder Phosphatiden, insbesondere Lecithin, zugemischt werden. Die Mono- und Diglyceride stammen vorzugsweise von den oben erwähnten Fettsäuretypen ab. Die Gesamtmenge an Schmier- und Trennmitteln beträgt vorzugsweise 0,1 bis 5 Gew.-%, bezogen auf das Gesamtgewicht der Masse für die jeweilige Schicht;

Fließmittel, z.B, Aerosil, in einer Menge von 0,1 bis 5 Gew.-%, bezogen auf das Gesamtgewicht des Gemisches;

Farbstoffe, wie Azofarbstoffe, organische oder anorganische Pigmente oder Farbstoffe natürlicher Herkunft, wobei anorganische Pigmente in einer Konzentration von 0,001 bis 10, vorzugsweise 0,5 bis 3 Gew.-%, bezogen auf das Gesamtgewicht des Gemisches bevorzugt sind;

Stabilisatoren, wie Antioxidanzien, Lichtstabilisatoren, Hydroperoxid-Vernichter, Radikalfänger, Stabilisatoren gegen mikrobiellen Befall.

Ferner können Netz-, Konservierungs-, Spreng-, Adsorptions-, Formentrenn- und Treibmittel zugesetzt werden (vgl. z.B. H. Sucker et al. Pharmazeutische Technologie, Thieme-Verlag, Stuttgart 1978).

Unter Hilfsstoffen im Sinne der Erfindung sind auch Substanzen zur Herstellung einer festen Lösung mit dem Wirkstoff zu verstehen. Diese Hilfsstoffe sind beispielsweise Pentaerythrit und Pentaerythrit-tetraacetat, Polymere wie z.B. Polyethylen- bzw. Polypropylenoxide und deren Blockcopolymere (Poloxamere), Phosphatide wie Lecithin, Homo- und Copolymere des Vinylpyrrolidons, Tenside wie Polyoxyethylen-40-stearat sowie Zitronen- und Bernsteinsäure, Gallensäuren, Sterine und andere wie z.B. bei J. L. Ford, Pharm. Acta Helv. 61, (1986), 69-88 angegeben.

Als Hilfsstoffe gelten auch Zusätze von Basen und Säuren zur Steuerung der Löslichkeit eines Wirkstoffes (siehe beispielsweise K. Thoma et al., Pharm. Ind. 51, (1989), 98-101).

Einzige Voraussetzung für die Eignung von Hilfsstoffen ist eine ausreichende Temperaturstabilität.

Wirkstoffe im Sinne der Erfindung sind alle Stoffe mit einer biologischen Wirkung, wie z.B. pharmazeutische Wirkstoffe, aber auch Vitamine und Mineralstoffe, sowie Pflanzenbehandlungsmittel und Insektizide.

Die Wirkstoffe dürfen sich unter den Verarbeitungsbedingungen nicht wesentlich zersetzen. Die Wirkstoffmenge pro Dosiseinheit und die Konzentration können je nach Wirksamkeit und Freisetzungsgeschwindigkeit in weiten Grenzen variieren. Die einzige Bedingung ist, dass sie zur Erzielung der gewünschten Wirkung ausreichen. So kann die Wirkstoffkonzentration im Bereich von 0,001 bis 95, vorzugsweise von 20 bis 80, insbesondere 30 bis 70 Gew.-% liegen. Auch Wirkstoff-Kombinationen können eingesetzt werden. Zu den Vitaminen gehören die Vitamine der A-Gruppe, der B-Gruppe, worunter neben $B_1$, $B_2$, $B_6$ und $B_{12}$ sowie Nicotinsäure und Nicotinamid auch Verbindungen mit Vitamin B-Eigenschaften verstanden werden, wie z.B. Adenin, Cholin, Pantothensäure, Biotin, Adenylsäure, Folsäure, Orotsäure, Pangamsäure, Carnitin, p-Aminobenzoesäure, myo-Inosit und Liponsäure sowie Vitamin C, Vitamine der D-Gruppe, E-Gruppe, F-Gruppe, H-Gruppe, I- und J-Gruppe, K-Gruppen und P-Gruppe. Zu Wirkstoffen im Sinne der Erfindung gehören auch Peptidtherapeutika und Impfstoffe.

Das erfindungsgemäße Verfahren ist beispielsweise zur Verarbeitung folgender Wirkstoffe bzw. der pharmakologisch aktiven Salze davon geeignet:

Acebutolol, Acetylcystein, Acetylsalicylsäure, Acyclovir, Alprazolam, Alfacalcidol, Allantoin, Allopurinol, Ambroxol, Amikacin, Amilorid, Aminoessigsäure, Amiodaron, Amitriptylin, Amlodipin, Amoxicillin, Ampicillin, Ascorbinsäure, Aspartam, Astemizol, Atenolol, Beclomethason, Benserazid, Benzalkonium-Hydrochlorid, Benzocain, Benzoesäure, Betamethason, Bezafibrat, Biotin, Biperiden, Bisoprolol, Bromazepam, Bromhexin, Bromocriptin, Budesonid, Bufexamac, Buflomedil, Buspiron, Coffein, Campher, Captopril, Carbamazepin, Carbidopa, Carboplatin, Cefachlor, Cefalexin, Cefadroxil, Cefazolin, Cefixim, Cefotaxim, Ceftazidim, Ceftriaxon, Cefuroxim, Selegilin, Chloramphenicol, Chlorhexidin, Chlor-pheniramin, Chlortalidon, Cholin, Cyclosporin, Cilastatin, Cimetidin, Ciprofloxacin, Cisapride, Cisplatin, Clarithromycin, Clävulansäure, Clomipramin, Clonazepam, Clonidin, Clotrimazol, Codein, Cholestyramin, Cromoglycinsäure, Cyanocobalamin, Cyproteron, Desogestrel, Dexamethason, Dexpanthenol, Dextromethorphan, Dextropropoxiphen, Diazepam, Diclofenac, Digoxin, Dihydrocodein, Dihydroergotamin, Dihydroergotoxin, Diltiazem, Diphenhydramin, Dipyridamol, Dipyron, Disopyramid, Domperidon, Dopamin, Doxycyclin, Enalapril, Ephedrin, Epinephrin, Ergacalciferol, Ergotamin, Erythromycin Estradiol, Ethinylestradiol, Etoposid, Eucalyptus Globulus, Famotidin, Felodipin, Fenofibrat, Fenoterol, Fentanyl, Flavin-Mononucleotid, Fluconazol, Flunarizin, Fluorouracil, Fluoxetin, Flurbiprofen, Furosemid, Gallopamil, Gemfibrozil, Gentamicin, Gingko Biloba, Glibenclamid, Glipizid, Clozapin, Glycyrrhiza glabra, Griseofulvin, Guaifenesin, Haloperidol, Heparin, Hyaluronsäure, Hydrochlorothiazid, Hydrocodon, Hydrocortison, Hydromorphon, Ipratropium-Hydroxid, Ibuprofen, Imipenem, Indomethacin, Iohexol, Iopamidol, Isosorbid-Dinitrat, Isosorbid-Mononitrat, Isotretinoin, Itraconazol, Ketotifen, Ketoconazol, Ketoprofen, Ketorolac, Labetalol, Lactulose, Lecithin, Levocarnitin, Levodopa, Levoglutamid, Levonorgestrel, Levothyroxin, Lidocain, Lipase, Imipramin, Lisinopril, Loperamid, Lorazepam, Lovastatin, Medroxyprogesteron, Menthol, Methotrexat, Methyldopa, Methylprednisolon, Metoclopramid, Metoprolol, Miconazol, Midazolam, Minocyclin, Minoxidil, Misoprostol, Morphin, Multivitamin-Mischungen bzw. -Kombinationen und Mineralsalze, N-Methylephedrin, Naftidrofuryl, Naproxen, Neomycin, Nicardipin, Nicergolin, Nicotinamid, Nicotin, Nicotinsäure, Nifedipin, Nimodipin, Nitrazepam, Nitrendipin, Nizatidin, Norethisteron, Norfloxacin, Norgestrel, Nortriptylin, Nystatin, Ofloxacin, Omeprazol, Ondansetron, Pancreatin, Panthenol, Pantothensäure, Paracetamol, Penicillin G, Penicillin V, Phenobarbital, Pentoxifyllin, Phenoxymethylpenicillin, Phenylephrin, Phenylpropanolamin, Phenytoin, Piroxicam, Polymyxin B, Povidon-Iod, Pravastatin, Prazepam, Prazosin, Prednisolon, Prednison, Bromocriptin, Propafenon, Propranolol, Proxyphyllin, Pseudoephedrin, Pyridoxin, Quinidin, Ramipril, Ranitidin, Reserpin, Retinol, Riboflavin, Rifampicin, Rutosid, Saccharin, Salbutamol, Salcatonin, Salicylsäure, Simvastatin, Somatropin, Sotalol, Spironolacton, Sucralfat, Sulbactam, Sulfamethoxazol, Sulfasalazin, Sulpirid, Tamoxifen, Tegafur, Teprenon, Terazosin, Terbutalin, Terfenadin, Tetracyclin, Theophyllin, Thiamin, Ticlopidin, Timolol, Tranexamsäure, Tretinoin, Triamcinolon-Acetonid, Triamteren, Trimethoprim, Troxerutin, Uracil, Valproinsäure, Vancomycin, Verapamil, Vitamin E, Folinsäure, Zidovudin.

Bevorzugte Wirkstoffe sind Ibuprofen (als Racemat, Enantiomer oder angereichertes Enantiomer), Ketoprofen, Flurbiprofen, Acetylsalicylsäure, Verapamil, Paracetamol, Nifedipin, Captopril, Omeprazol, Ranitidin, Tramadol, Cyclosporin, Trandolapril und Peptidtherapeutika.

Im Einzelnen kann es zur Ausbildung von festen Lösungen kommen. Der Begriff "festen Lösungen" ist dem Fachmann geläufig, beispielsweise aus der eingangs zitierten Literatur. In festen Lösungen von Wirkstoffen in Polymeren liegt der Wirkstoff molekulardispers im Polymer vor.

Vorzugsweise liegt die Masse der hergestellten wirkstoffhaltigen Formen zwischen 50 mg und 2000 mg, besonders bevorzugt zwischen 150 mg und 1300 mg.

Das erfindungsgemäße Verfahren weist dieselben Vorteile wie die erfindungagemäße Vorrichtung auf. Insbesondere ist es bei dem erfindungsgemäßen Verfahren möglich, die wirkstoffhaltige Formulierung kontinuierlich zu plastifizieren und gleichzeitig die wirkstoffhaltige Form durch einen Einspritzvorgang zu erzeugen. Dies wird dadurch ermöglicht, daß die plastifizierte Formulierung nicht direkt in ein Formwerkzeug eingespritzt wird oder nur einer Spritzeinheit zugeführt wird, sondern zumindest zwei Spritzeinheiten.

Die Erfindung wird nun anhand von Ausführungsbeispielen unter Bezugnahme auf die beigefügten Zeichnungen näher erläutert.

Figur 1 zeigt eine besonders bevorzugte Ausführungsform der Vorrichtung zur Durchführung des erfindungsgemäßen Verfahrens,

Figur 2 zeigt eine weitere Ausführungsform einer Vorrichtung, bei der eine Speichereinheit 4 zwischen dem Extruder 1 und der Spritzeinheit 2 angeordnet ist,

Figur 3 zeigt die in Figur 1 gezeigte Ausführungsform in einer allgemeineren Ausgestaltung,

Figur 4 zeigt eine weitere Ausführungsform der Vorrichtung,

Figur 4a zeigt Wirkstofffreisetzungsprofile 0. Ordnung und nicht 0. Ordnung,

Figur 5 zeigt ein Beispiel eines Heißkanalsystems und

Figur 6 zeigt eine PVT-Messung einer Verapamil-Rezeptur.

Die Vorrichtung dient der Herstellung von festen wirkstoffhaltigen Formen, die eine wirkstoffhaltige Formulierung enthalten, die mindestens ein geeignetes, z.B. wasserlösliches oder in Wasser quellbares polymeres Bindemittel enthält. Die Ausgangsstoffe der Formulierung werden beispielsweise als pulverförmige Stoffe bzw. Stoffgemische einem Extruder 1 zugeführt. In den Extruder wird die eingefüllte Formulierung kontinuierlich plastifiziert, d.h. in einen hinreichend fließfähigen Zustand überführt. Normalerweise erfolgt dies über eine Erwärmung der in dem Extruder 1 enthaltenen Formulierung. Des weiteren werden die Ausgangsstoffe in dem Extruder 1 gemischt und homogenisiert.

Der Extruder 1 kann beispielsweise ein Doppelschneckenextruder sein, der zwei nebeneinanderliegende Schnecken aufweist, die in entgegengesetzter oder gleicher Drehrichtung rotieren. Ferner kann der Extruder 1 auch ein Mehrschneckenextruder, ein Kneter, ein Doppel- oder Mehrschneckenextruder mit Schmelzpumpe, ein Doppel- oder Mehrschneckenextruder mit Entgasungseinrichtung oder ein Planetwalzenextruder sein. Unter Umständen wäre auch die Verwendung eines Einschneckenextruders möglich, wobei in diesem Fall jedoch Pulvermischungen nur eingeschränkt verarbeitet werden können.

Mit dem Extruder 1 sind in der in Figur 1 gezeigten Ausführungsform zwei getrennt voneinander vorgesehene Spritzgießeinheiten $2_1$ und $2_2$ verbunden, so daß die plastifizierte Formulierung von dem Extruder 1 in die Spritzeinheiten $2_1$ und $2_2$ gelangt. Vorteilhafterweise erfolgt die Beschickung der Spritzeinheiten $2_1$ und $2_2$ über ein Zweiwegeventil 5, dem die plastifizierte Formulierung von dem Extruder 1 zugeführt wird und das die plastifizierte Formulierung auf die beiden Spritzeinheiten $2_1$ und $2_2$ verteilt. Dabei erfolgt die Beschikkung der Spritzeinheiten 2 wechselweise. Das Verteilerventil 5 sollte möglichst kurze Schaltzeiten besitzen.

Die Spritzeinheiten 2 sind in der hier beschriebenen Ausführungsform Kolbeneinspritzeinheiten. Bei einer Kolbeneinspritzeinheit befindet sich ein Spritzkolben innerhalb eines Spritzzylinders. Der Spritzkolben drückt gegen die in dem Spritzzylinder befindliche plastifizierte Formulierung, so daß diese durch die Ausgangsöffnung der Spritzeinheit 2 austritt und in ein Formwerkzeug gelangen kann.

Vorteilhafterweise entspricht das Volumen des Zylinders einer Spritzeinheit 2 dem Volumen des mit der Spritzeinheit 2 zu beschickenden Formwerkzeugs 3 oder, falls bei einem Spritzvorgang mehrere Formwerkzeuge 3 beschickt werden sollen, dem Volumen dieser Formwerkzeuge 3. Dies bedeutet, daß sich nach jedem Spritzvorgang keine wirkstoffhaltige Formulierung mehr in der Spritzeinheit 2 befindet.

Jede Spritzeinheit 2 kann ein bestimmtes Formulierungsvolumen in zumindest ein Formwerkzeug 3 einspritzen. In der in Figur 1 gezeigten Ausführungsform ist für die Spritzeinheiten $2_1$ und $2_2$ jeweils ein Formwerkzeug $3_1$ und $3_2$ vorgesehen. In diesem Fall kann über das Verteilerventil 5 die plastifizierte Formulierung zunächst in die Spritzeinheit $2_1$ gelangen. Ist die Spritzeinheit $2_1$ vollständig gefüllt, kann die von dem Extruder 1 kontinuierlich erzeugte plastifizierte Formulierung über das Verteilerventil 5 in die zweite Spritzeinheit $2_2$ gelangen. Währenddessen spritzt die erste Spritzeinheit 21, wie in ihr enthaltene Formulierung in das Formwerkzeug $3_1$ ein. Wenn der Einspritzvorgang der ersten Spritzeinheit $2_1$ abgeschlossen ist, d.h. wenn die erste Spritzeinheit $2_1$ erneut ein bestimmtes Formulierungsvolumen aufnehmen kann, ist vorteilhafterweise das Befüllen der zweiten Spritzeinheit $2_2$ gerade abgeschlossen, so daß direkt daran anschließend erneut die erste Spritzeinheit $2_1$ gefüllt werden kann.

Demgemäß ist es möglich, daß der Extruder 1 kontinuierlich betrieben wird und über das Verteilerventil 5 von den nur diskontinuierlich arbeitenden Spritzeinheiten 2 entkoppelt ist.

In einer weiteren Ausgestaltung besteht das zu einer Spritzeinheit 2 gehörende Formwerkzeug 3 aus mehreren parallel geschalteten Einzelwerkzeugen $3_1$ bis $3_n$. Bei einem Einspritzvorgang wird die Formulierung von der Spritzeinheit 2 gleichzeitig in die parallel geschalteten Einzelformwerkzeuge $3_1$ bis $3_n$ eingespritzt. Des weiteren ist es möglich, daß das Formwerkzeug aus mehreren, einzeln an die Spritzeinheit 2 ankoppelbaren Formwerkzeugen $3_1$ bis $3_n$ besteht, in die die Formulierung nacheinander eingespritzt werden kann. Hierzu ist vorteilhafterweise eine automatisch betriebene Austauschvorrichtung vorgesehen, die die einzelnen Formwerkzeuge $3_1$ bis $3_n$ nacheinander an die ihnen zugeordnete Spritzeinheit 2 ankoppelt.

Ferner ist es möglich, daß für mehrere Spritzeinheiten 2 jeweils ein Formwerkzeug 3 vorgesehen ist, das wechselweise an die Spritzeinheiten 2 ankoppelbar ist. Diese Ausgestaltung sollte dann verwendet werden, wenn die Schmelzeplastifizierung in dem Extruder 1 der geschwindigkeitsbestimmende Schritt ist.

Figur 3 zeigt eine weitere Ausführungsform des Gerätes, die eine Verallgemeinerung der in Figur 1 gezeigten Ausführungsform ist. Bei dieser Ausführungsform ist der Extruder 1 mit einem $m \otimes k$-fachen verteilerventil 5 verbunden. Dieses Verteilerventil 5 verteilt die in dem Extruder 1 kontinuierlich erzeugte Formulierung auf $m \otimes k$ Spritzgießeinheiten 2. In Figur 3 sind die Spritzeinheiten 2 so bezeichnet, daß der Index k die Menge der Spritzeinheiten 2 angibt, die ein Formwerkzeug 3 oder eine Gruppe von Formwerkzeugen beschicken. Der Index m gibt die Anzahl der Gruppen von Spriteinheiten an, welche die m Formwerkzeuge 3 oder Gruppen von Formwerkzeugen beschicken. Ferner bezeichnet der Index n wieviele Werkzeuge 3 innerhalb einer Gruppe von Werkzeugen einer Gruppe von Spritzgießeinheiten 2 mit gegebenem Index m zugeordnet sind. Selbstverständlich sind die Indizes m, k, n ganzzahlig und größer 1. Des weiteren gilt für diese Ausführungsform der erfindungsgemäßen Vorrichtung, daß $m + k > 2$ immer erfüllt ist. Dies bedeutet, daß zumindest zwei Spritzgießeinheiten 2 vorgesehen sein müssen.

Bei dieser allgemeineren Ausgestaltung des Gerätes kann das $m \otimes k$-fache Verteilerventil 5 wie bei der in Bezug auf Figur 1 erläuterten Ausführungsform die Spritzeinheiten 2 nacheinander befüllen.

Eine weitere Ausbildung der zuvor erläuterten Ausführungsformen des Gerätes ist in Figur 2 gezeigt. Bei dieser Ausgestaltung ist bei der Verbindung des Extruders 1 zu einer oder mehreren Spritzgießeinheit(en) 2 eine Abzweigung vorgesehen, die zu einer Speichereinheit 4 führt. Selbstverständlich kann zusätzlich zwischen dem Extruder 1 und den Spritzeinheiten 2 das Verteilerventil 5 vorgesehen sein. Dies ist jedoch nicht unbedingt erforderlich. Die Speichereinheit 4 dient auch der Entkopplung des vorteilhafterweise kontinuierlich betriebenen Extruders 1 von den nur diskontinuierlich betreibbaren-Spritzeinheiten 2. Die Speichereinheit 4 kann die während eines Einspritzvorgangs plastifizierte Formulierung zwischenspeichern und anschließend die zwischengespeicherte Formulierung schonend und schnell an eine oder die Spritzeinheiten 2 weiterleiten. Hierzu weist vorteilhafterweise die Speichereinheit 4 auch eine Fördereinrichtung auf.

Bei einem Spezialfall des in Figur 2 gezeigten Ausführungsbeispiels ist der Index k gleich i, d.h. es ist ein Extruder 1 mit nur einer Spritzeinheit 2 versehen. In diesem Fall kann, obwohl die Extrusion kontinuierlich erfolgt und die Spritzeinheit 2 diskontinuierlich arbeitet, die Vorrichtung aufgrund der Schmelzespeicherung in der Speichereinheit 4 kontinuierlich betrieben werden.

In einer weiteren Ausführungsform der Vorrichtung weist diese mehrere Extruder 1 zum Verarbeiten unterschiedlicher Massen auf, wobei zumindest eine Masse eine wirkstoffhaltige Formulierung ist. Figur 4 zeigt ein Bespiel für diese Ausführungsform mit zwei Extrudern $1_1$ und $1_2$. Beide Extruder $1_1$ und $1_2$ sind jeweils mit Spritzeinheiten $2_1$ bzw. $2_2$ verbunden. Ferner sind die spritzeinheiten $2_1$ bzw. $2_2$ mit Formwerkzeugen 3 verbunden. Der Aufbau einer zu einem Extruder 1 gehörenden Einheit ist identisch zu dem der vorstehend mit Bezug zu Figur 3 bzw. Figur 2 erläuterten Ausführungsformen mit einem Extruder 1. Die in Figur 4 gezeigte Ausführungsform der Vorrichtung ist **dadurch gekennzeichnet, daß** ein Formwerkzeug 3 mit zumindest zwei Spritzeinheiten $2_1$ und $2_2$ in Verbindung steht, denen Massen von verschiedenen Extrudern $1_1$ und $1_2$ zugeführt werden. Damit kann mit einer Vorrichtung gemäß dieser Ausführungsform ein Mehrkomponentenspritzguß durchgeführt werden. Es können somit Mehrkomponenten-Formkörper, wie z.B. direkt magensaftresistent gecoatete Tabletten, mehrfach gecoatete Tabletten, Mehrschichttabletten mit einem z.B. mehrphasigen Freisetzungsprofil oder Formen mit einem Freisetzungsprofil 0. Ordnung hergestellt werden. Solche Formen sind **dadurch gekennzeichnet, daß** die aktive Oberfläche der Form während der Auflösung der Form konstant bleibt und somit auch die freisetzende Wirkstoffmenge pro Zeiteinheit. Ein daraus resultierendes Freisetzungsprofil 0. Ordnung ist in Figur 4a dargestellt. Mögliche Formen mit einem Freisetzungsprofil 0. Ordnung wurden bereits von W.D. Rhine et al., in "Controlled Release of Bioactive Materials, Academic Press, 1980, ISBN 0-12-074450-3 beschrieben.

Die herzustellende wirkstoffhaltige Form kann z.B. im wesentlichen halbkugelförmig mit einer konkaven Vertiefung in der Mitte der planaren Seite der Halbkugel sein (d.h. die Form einer halben Kugel mit Hohlraum haben) oder die Hälfte einer dickwandigen Hohlkugel. Des weiteren kann die herzustellende Form ein halber Hohlzylinder sein. Um ein möglichst lineares Freisetzungsprofil zu erhalten, sollten die Formen so durch das Mehrschichtverfahren hergestellt sein, dass der Wirkstoff nur über die Innenwandungen der halben Hohlkörper abgegeben werden kann, wie es im vorstehend erwähnten Artikel von W.D. Rhine et al. beschrieben wurde.

In einer weiteren bevorzugten Ausgestaltung der zuvor erläuterten Vorrichtungen werden die Spritzeinheiten und die Formwerkzeuge über Heißkanalsysteme verbunden, deren Verwendung sowohl eine höhere Ausbeute als auch eine Vergrößerung der Produktionsrate mit sich bringt, da der beim konventionellen Spritzguß mit erstarrende und meist die Zykluszeit bestimmende Anguß entfällt. Besonders für Spritzgussprozesse, in denen der prozentuale Anteil des Angusses an der Gesamtmasse sehr hoch ist und dieser aufgrund der mehrfachen thermischen Belastung nicht wieder als Rohstoff eingesetzt werden kann und für die große Stückzahlen gefordert werden (z.B. Herstellung von Tabletten) wird durch die Verwendung von Heißkanalsystemen die Wirtschaftlichkeit des Prozesses erheblich erhöht. Figur 5 zeigt ein solches Heißkanalsystem. Die plastifizierte Formulierung tritt bei Bezugszeichen 11 in einen Heißkanalverteiler 10 ein und tritt bei 12 aus den Heißkanaldüsen 7 des Heißkanalsystems aus, um in die Formwerkzeuge 3 zu gelangen. In der in Figur 5 gezeigten besonders bevorzugten Ausführungsform erfolgt die Temperatursteuerung oder -regelung über ein Fluid, das in den Heißkanalverteiler 10 bei Bezugszeichen 8a eintritt und bei Bezugszeichen 8b austritt. Des weiteren tritt das Temperierfluid für die Heißkanaldüsen 7 bei Bezugszeichen 9a ein und bei Bezugszeichen 9b aus. Die Formulierung gelangt über die Einspritzkanäle 6, die in den Heißkanaldüsen 7 vorgesehen sind, zu den Formwerkzeugen 3.

Eine Fluidtemperierung des Heißkanalsystems besitzt den Vorteil, daß die durch Kompression und innere Reibung während des Einspritzvorgangs entstehende Wärme sehr gut abgeführt werden kann. Außerdem kann es bei einer Fluidtemperierung im Gegensatz zur elektrischen Beheizung, bei der sich in Bereichen, die jeweils näher zu elektrischen Heizelementen liegen, zu hohe Temperaturen einstellen, zu keiner lokalen Überhitzung der Formulierung und somit zu einer Schädigung der in der Formulierung enthaltenen Wirkstoffe kommen. Aus diesem Grunde ist auch für die Temperatursteuerung oder -regelung der Spritzeinheiten 2, der Auslaßöffnung des Extruders 1 sowie des Verteilerventils 5 die Verwendung einer Fluidtemperierung vorteilhaft.

Erfolgt die Temperatursteuerung oder regelung des Heißkanalsystems über eine Elektrobeheizung kann das Heißkanalsystem mit einer geeigneten Gegenkühlung versehen werden, um die aufgrund von Kompression und innerer Reibung entstehende Wärme besser abführen zu können und somit eine Überhitzung und eine eventuelle Schädigung des in der Formulierung enthaltenen Wirkstoffs zu vermeiden.

Durch die Verwendung eines Heißkanalsystems werden die wirkstoffhaltigen Formen nach der Abkühlung ohne die Anhängsel der abgekühlten Formulierung, die im Einspritzkanal verblieben sind, ausgeworfen, da die Formulierung in dem Einspritzkanal 6 durch dessen Temperierung bei einer Temperatur gehalten werden kann, die ein Erstarren der Formulierung und damit eine Verbindung zu der abgekühlten wirkstoffhaltigen Form verhindert.

Wird für das Heißkanalsystem eine Fluidtemperierung bzw. eine Elektrotemperierung mit Gegenkühlung verwendet, kann eine aufgrund von internen Effekten wie z.B. Kompression oder innerer Reibung entstehenden Temperaturerhöhung der Formulierung verhindert werden. Eine solche Temperaturerhöhung kann zu einer Zersetzung von Bestandteilen der wirkstoffhaltigen Formulierung führen.

Wird das Heißkanalsystem mit einer Fluidtemperierung versehen, kann außerdem eine lokale Überhitzung der Formulierung verhindert werden.

Die Temperatur des erfindungsgemäßen Heißkanalsystems liegt überlicherweise zwischen 50 und 250°C, meist jedoch zwischen 100 und 200°C.

Eine Temperaturerhöhung $\Delta T$ erfährt die Formulierung durch Kompression. Die Temperaturerhöhung $\Delta T$ berechnet sich nach folgender Formel:

$$\Delta T = \frac{\alpha T}{\varrho c_p} \Delta p ,$$

wobei a der isobare Volumenausdehnungskoeffizient, T die Temperatur der Formulierung, $\ell$ die Dichte, cp die spezifische Wärmekapazität bei konstantem Druck und $\Delta p$ die plötzliche Druckerhöhung bei einer adiabatischen Kompression bezeichnen. Die Werte für $\alpha$ und $\ell$ bei einem bestimmten Verarbeitungsdruck und einer bestimmten Verarbeitungstemperatur können z.B. pVT-Diagrammen entnommen werden.

Beispiel:

Durch die Kompression einer Schmelze bestehend aus 48,0% Verapamil-HCl, 31,5% Hydroxypropylcellulose Typ EF, 17,5% Methylhydroxypropylcellulose Typ V3000 und 3,0% Lecithin von $1.10^5$ Pa auf $1{,}4\cdot10^8$ Pa erhält man mit $\alpha = 6{,}466\cdot10^{-4}\ K^{-1}$, $\varrho = 1115\ kg/m^3$ (Werte aus pVT-Diagramm für obige Verapamil-Rezeptur gemäß Figur 6) und $c_p = 2406\ J\ kg^{-1}\ K^{-1}$ bei einer Verarbeitungstemperatur von T = 126°C eine Erhöhung der Schmelzetemperatur von $\Delta\overline{T}$= 13,5°C.

Eine weitere Temperaturerhöhung erfährt die plastifizierte Formulierung durch Strömungsvorgänge während des Einströmens in die Kavitäten des Formwerkzeugs aufgrund innerer Reibung. Bei Vernachlässigung des Wärmeaustauschs über die Wandungen des Schmelze-Verteilersystems berechnet sich die mittlere Temperaturerhöhung $\Delta\overline{T}$ im Düse-Angußsystem DA zu

$$\Delta\overline{T} = \frac{\Delta p_{DA}}{\varrho c_p}$$

Beispiel:

Bei Verarbeitung der oben bereits aufgeführten Verapamil-Rezeptur erhält man mit der obigen Formel für eine Verarbeitungstemperatur von T = 126°C und $\Delta_{PDA} = 1{,}399\cdot10^8$ Pa, $\varrho = 1115\ kg/m^3$ und $c_p = 2406\ J\ kg^{-1}\ K^{-1}$ eine Schmelzetemperaturerhöhung von $\Delta\overline{T}$= 52,1°C.

Solche Temperaturerhöhungen der Formulierung führen bei vielen Formulierungen zu einer Schädigung des Wirkstoffs. Wird die Formmulierung über ein Heißkanalsystem auf die einzelnen Kavitäten des Formwerkzeugs verteilt, kann durch den Einsatz des besonders bevorzugten erfindungsgemäßen, fluidtemperierten Heißkanalsystems bzw. durch ein elektrisch beheiztes Heißkanalsystem mit Gegenkühlung eine Schädigung des Wirkstoffs durch eine durch Kompression und innere Reibung im Heißkanal hervorgerufene Temperaturerhöhung verhindert werden, da die entstehende Wärme gut abgeführt werden kann. Durch eine Fluidtemperierung können zusätzlich noch lokale Überhitzungen in der Formulierung vermieden werden.

Im Folgenden wird ein Verfahrensbeispiel für die Herstellung einer festen wirkstoffhaltigen Form aus einer wirkstoffhaltigen Formulierung angegeben. Zur Durchführung des Verfahrens wird die in Figur 1 dargestellte Vorrichtung verwendet. Dabei wurde als Extruder der Doppelschneckenextruder ZSK 25 der Firma Werner & Pfleiderer verwendet. Die Spritzgießeinheiten 2 waren Kolbeneinspritzeinheiten, die flüssig temperiert waren. Die Formwerkzeuge $3_1$ und $3_2$ waren jeweils ein 4-fach Einzelwerkzeug mit Formkörper. Als Schließeinheiten für die Formwerkzeuge wurden die Einheiten Arburg 270 S (ohne Plastifizier- und Einspritzeinheit) verwendet.

Für die wirkstoffhaltigen Formen wurde die Verapamil-Rezeptur (48,0% Verapamil-HCl, 31,5% Hydroxypropylcellulose Typ EF, 17,5% Methylhydroxypropylcellulose Typ V3000, 3,0% Lecithin) verwendet.

Die Schmelztemperatur war 126°C, die Zykluszeit 9 s, der Spritzdruck 1400 bar, die Einzelmasse pro Formkörper 430 mg und der Durchsatz durch den Extruder 1 war 1,3,8 kg/h. Die Schmelzeverteilung in die beiden Formwerkzeuge $3_1$ und $3_2$ erfolgte jeweils mittels eines Heißkanalsystems, welches mit jeweils vier Heißkanaldüsen mit beheiztem Einspritzkanal ausgestattet war.

Während der Versuchsdurchführung wurden von den hergestellten Formkörpern Proben entnommen und deren Gehalt an Verapamil-EC1 und zersetzungsprodukten mittels HPLC (Hochleistungsflüssigkeitschromatographie) bestimmt. Die Ergebnisse sind in der folgenden Tabelle zusammengefaßt:

| | Gehalt Verapamil-HCl [%] | Gehalt Zersetzungsprodukt 1 (rRT=0,56) [%] | Gehalt Zersetzungsprodukt 2 (rRT=1,46) [%] |
|---|---|---|---|
| Probe 1 | 47,8 | 0,01 | < 0,01 |
| Probe 2 | 47,4 | 0,02 | < 0,01 |

Die Werte zeigen, daß der Wirkstoff weder durch die Plastifizierung im Doppelschneckenextruder noch durch den Spritzgießprozeß im größeren Maße geschädigt wurde.

Zusätzlich wurde die Kristallinität der Proben mittels DSC (Differential Scanning Calorimetry) bestimmt.

| | Kristallinität [%] |
|---|---|
| Probe 1 | 0 |
| Probe 2 | 0 |

Die Ergebnisse der DSC-Messungen zeigen, daß der Wirkstoff zu 100% molekulardispers in der Matrix vorliegt.

Es wird bemerkt, daß zuvor beschriebene Ausgestaltungen von Ausführungsformen der Erfindung auch in Kombination mit anderen Ausführungsformen der Erfindung verwendet werden können.

## Patentansprüche

**1.** Verfahren zum Herstellen von festen wirkstoffhaltigen Formen aus einer wirkstoffhaltigen Formulierung, die mindestens ein polymeres Bindemittel enthält, bei dem

- die wirkstoffhaltige Formulierung kontinuierlich plastifiziert wird,
- die plastifizierte Formulierung einer Spritzeinheit ($2_1$) zugeführt wird, die in dieser Spritzeinheit ($2_1$) befindliche Formulierung in ein Formwerkzeug ($3_1$) eingespritzt wird, und während des Einspritzens in das eine Fomwerkzeug ($3_1$) durch die eine Spritzeinheit ($Z_1$) die plastifizierte Formulierung einer weiteren Spritzeinheit ($2_2$) zugeführt wird und die in der weiteren Spritzeinheit ($2_2$) befindliche Formulierung in das Formwerkzeug ($3_1$) oder ein anderes Formwerkzeug ($3_2$) eingespritzt wird, wobei das Befüllen der weiteren Spritzeinheit ($2_2$) gerade abgeschlossen ist, wenn der Einspritzvorgang der einen Spritzeinheit ($2_1$) abgeschlossen ist und die eine Spritzeinheit ($2_1$) erneut ein Formulierungsvolumen aufnehmen kann,
- die Temperatur der plastifizierten Formulierung mittels eines Heißkanalsystems (7, 10) mit beheizten Einspritzkanälen (6) gesteuert oder geregelt wird, wobei das Heißkanalsystem zur Temperatursteuerung oder -regelung der Einspritzkanäle (6) ein Fluid oder eine Elektroheizung mit Gegenkühlung aufweist.

**2.** Verfahren gemäß Anspruch 1, **dadurch gekennzeichnet, dass** die Temperatur der plastifizierten Formulierung auf einen Bereich zwischen 50 und 250 °C gesteuert oder geregelt wird.

**3.** Verfahren gemäß Anspruch 1 oder 2, **dadurch gekennzeichnet, dass** das einer Spritzeinheit ($2_1$, $2_2$) ugeführte Formulierungsvolumen dem in das Formwerkzeug ($3_1$, $3_2$) eingespritzte Volumen entspricht.

**4.** Verfahren gemäß Anspruch 1, 2 oder 3, **dadurch gekennzeichnet, dass** vor dem Einspritzen zwei Folien in das Formwerkzeug ($3_1$, $3_2$) eingelegt werden, zwischen die die Formulierung eingespritzt wird.

## Claims

**1.** Process for producing solid forms containing active substance from a formulation containing active substance which comprises at least one polymeric binder, wherein

- the formulation containing active substance is plasticised continuously,
- the plasticised formulation is supplied to an injection unit ($2_1$), the formulation contained in this injection unit ($2_1$) is injected into a moulding tool ($3_1$), and during the injection into the moulding tool ($3_1$) through the injection unit ($2_1$) the plasticised formulation is supplied to a further injection unit ($2_2$) and the formulation contained in the further injection unit ($2_2$) is injected into the moulding tool ($3_1$) or another moulding tool ($3_2$), the filling of the further injection unit ($2_2$) having just finished when the injection process of one injection unit ($2_1$) has ended and the injection unit ($2_1$) can receive another volume of formulation,
- the temperature of the plasticised formulation is controlled or regulated by means of a hot channel system (7, 10) with heated injection channels (6), the hot channel system comprising a fluid or electric heating means with counter-cooling for controlling or regulating the temperature of the injection channels (6).

2. Process according to claim 1, **characterised in that** the temperature of the plasticised formulation is controlled or regulated to a range between 50 and 250°C.

3. Process according to claim 1 or 2, **characterised in that** the volume of formulation supplied to an injection unit ($2_1$, $2_2$) corresponds to the volume injected into the moulding tool ($3_1$, $3_2$).

4. Process according to one of claims 1, 2 or 3, **characterised in that** before the injection two films are placed in the moulding tool ($3_1$, $3_2$) and the formulation is injected between them.

## Revendications

1. Procédé pour produire des corps moulés solides contenant un ou des principes actifs à partir d'une formulation contenant un ou des principes actifs qui contient au moins un liant polymère, dans lequel :

   - la formulation contenant un ou des principes actifs est plastifiée en continu,
   - la formulation plastifiée est envoyée à une unité d'injection ($2_1$), la formulation qui se trouve dans cette unité d'injection ($2_1$) est injectée dans un outil de moulage ($3_1$), et pendant l'injection dans l'outil de moulage ($3_1$) par l'unité d'injection ($2_1$), la formulation plastifiée est envoyée à une autre unité d'injection ($2_2$) et la formulation qui se trouve dans l'autre unité d'injection ($2_2$) est injectée dans l'outil de moulage ($3_1$) ou dans un autre outil de moulage ($3_2$), où le remplissage de l'autre unité d'injection ($2_2$) est juste terminé quand le processus d'injection de l'unité d'injection ($2_1$) est terminé et l'unité d'injection ($2_1$) peut recevoir de nouveau un volume de formulation,
   - la température de la formulation plastifiée est commandée ou régulée au moyen d'un système à canaux chauds (7, 10) avec des canaux d'injection (6) chauffés, où le système à canaux chauds comporte un fluide ou un chauffage électrique avec contre-refroidissement pour la commande ou régulation de la température des canaux d'injection (6).

2. Procédé selon la revendication 1 **caractérisé en ce que** la température de la formulation plastifiée est commandée ou régulée dans un domaine entre 50 et 250°C.

3. Procédé selon la revendication 1 ou 2 **caractérisé en ce que** le volume de formulation envoyé à une unité d'injection ($2_1$, $2_2$) correspond au volume injecté dans l'outil de moulage ($3_1$, $3_2$).

4. Procédé selon la revendication 1, 2 ou 3 **caractérisé en ce que**, avant l'injection, deux feuilles entre lesquelles la formulation est injectée sont disposées dans l'outil de moulage ($3_1$, $3_2$).

Figur 1

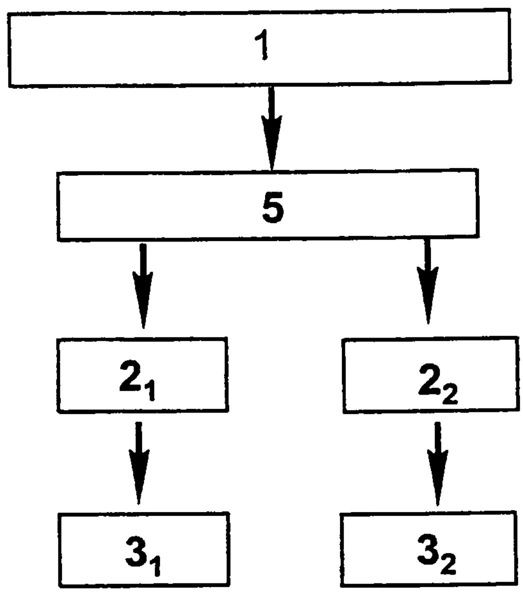

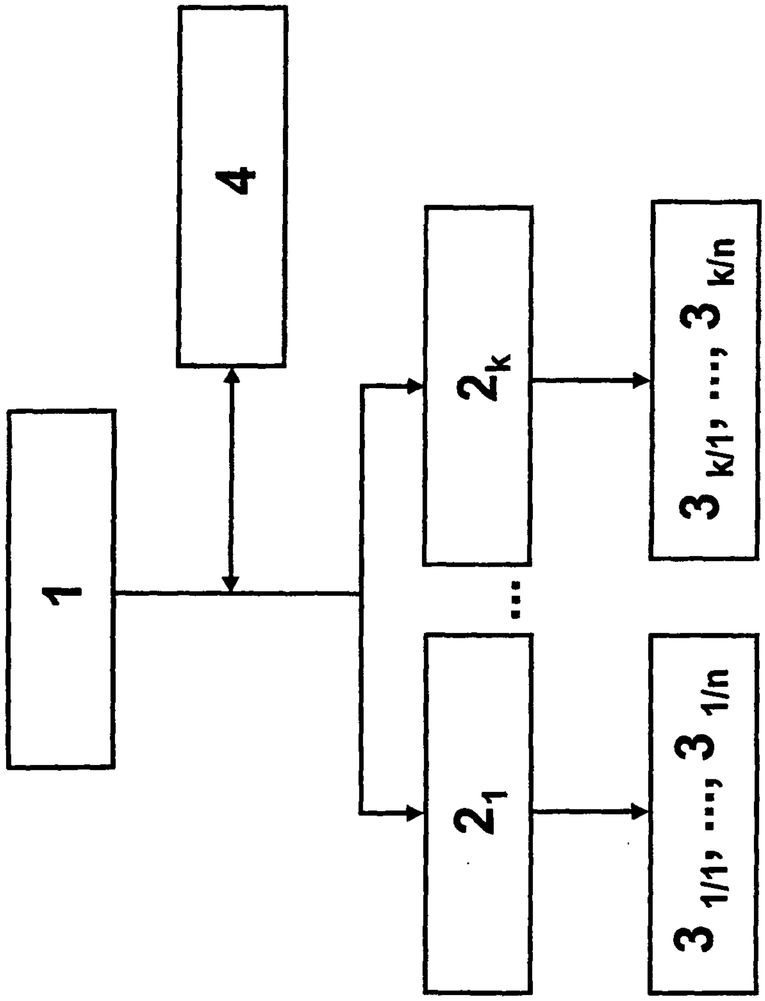


Figur 2

Figur 3

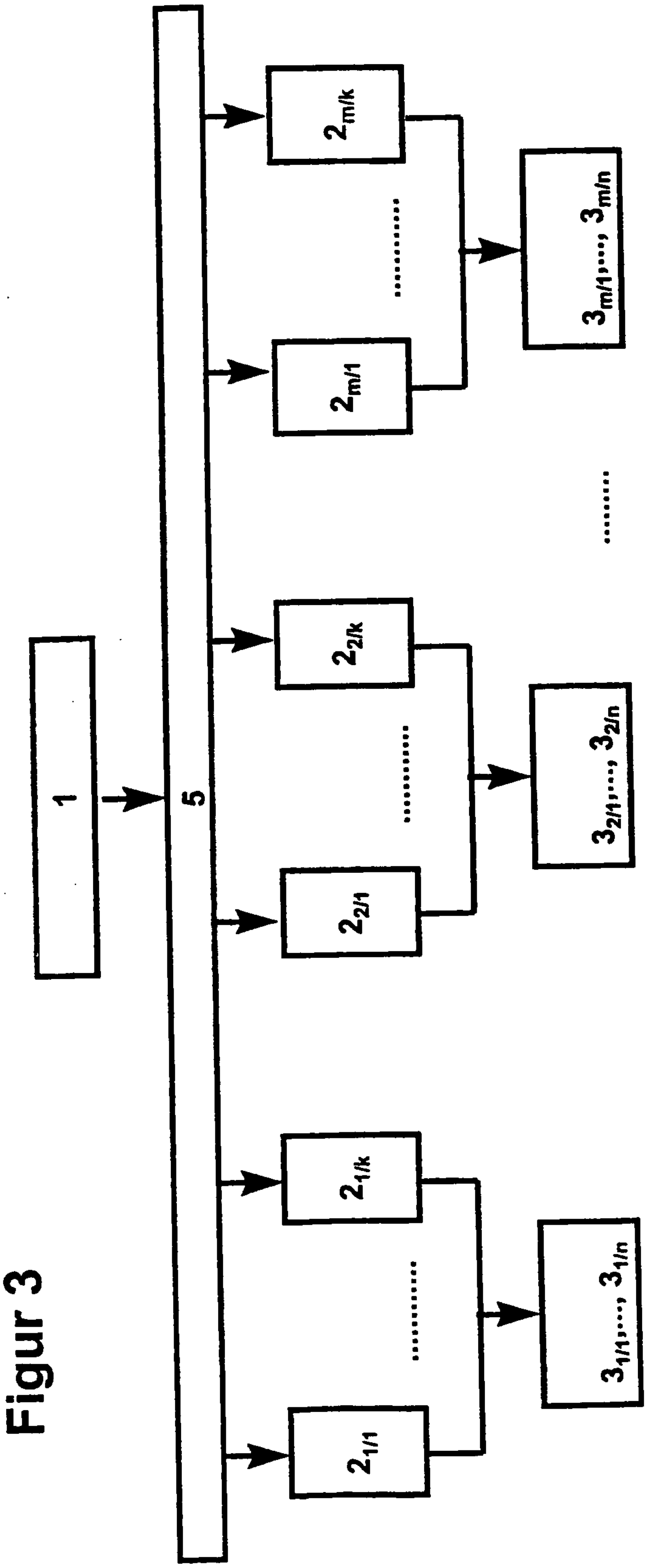

m=1,2,...
k=1,2,...
n=1,2,...
m+k>=3

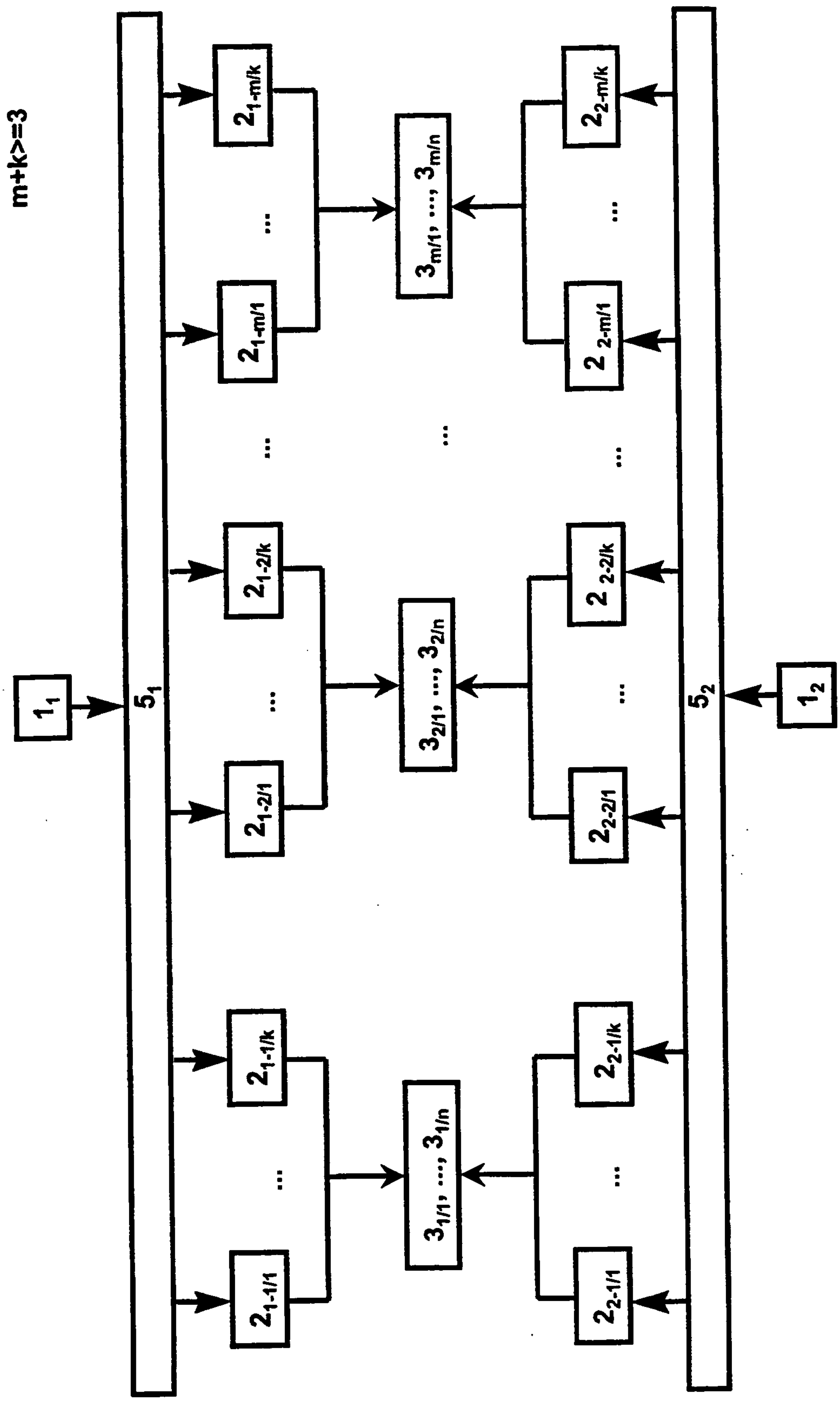


Figur 4

Figur 4a

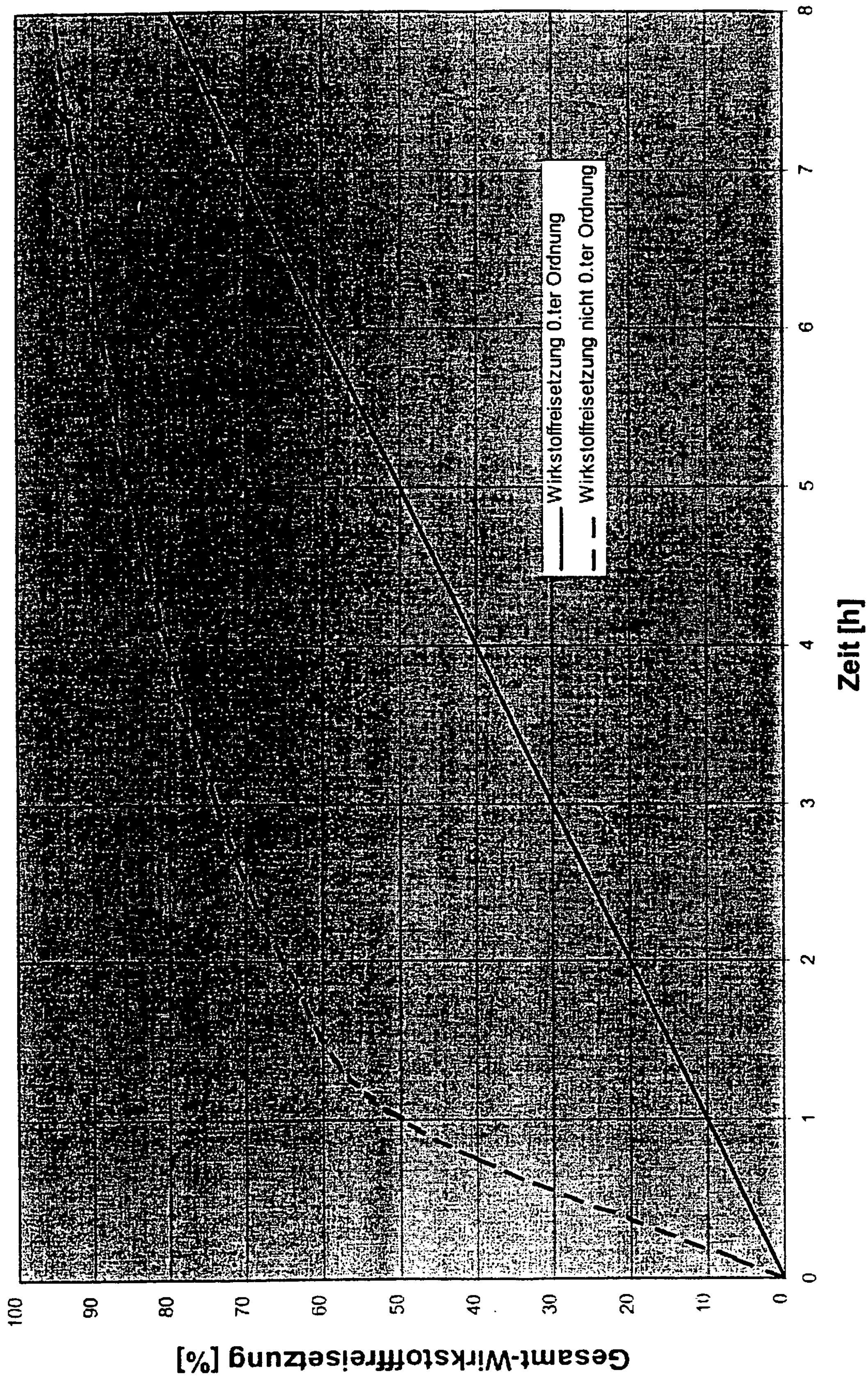
Gesamt-Wirkstofffreisetzung [%]
100
90
80
70
60
50
40
30
20
10
0
0
1
2
3
4
5
6
7
8
Zeit [h]
Wirkstoffreisetzung 0.ter Ordnung
Wirkstoffreisetzung nicht 0.ter Ordnung

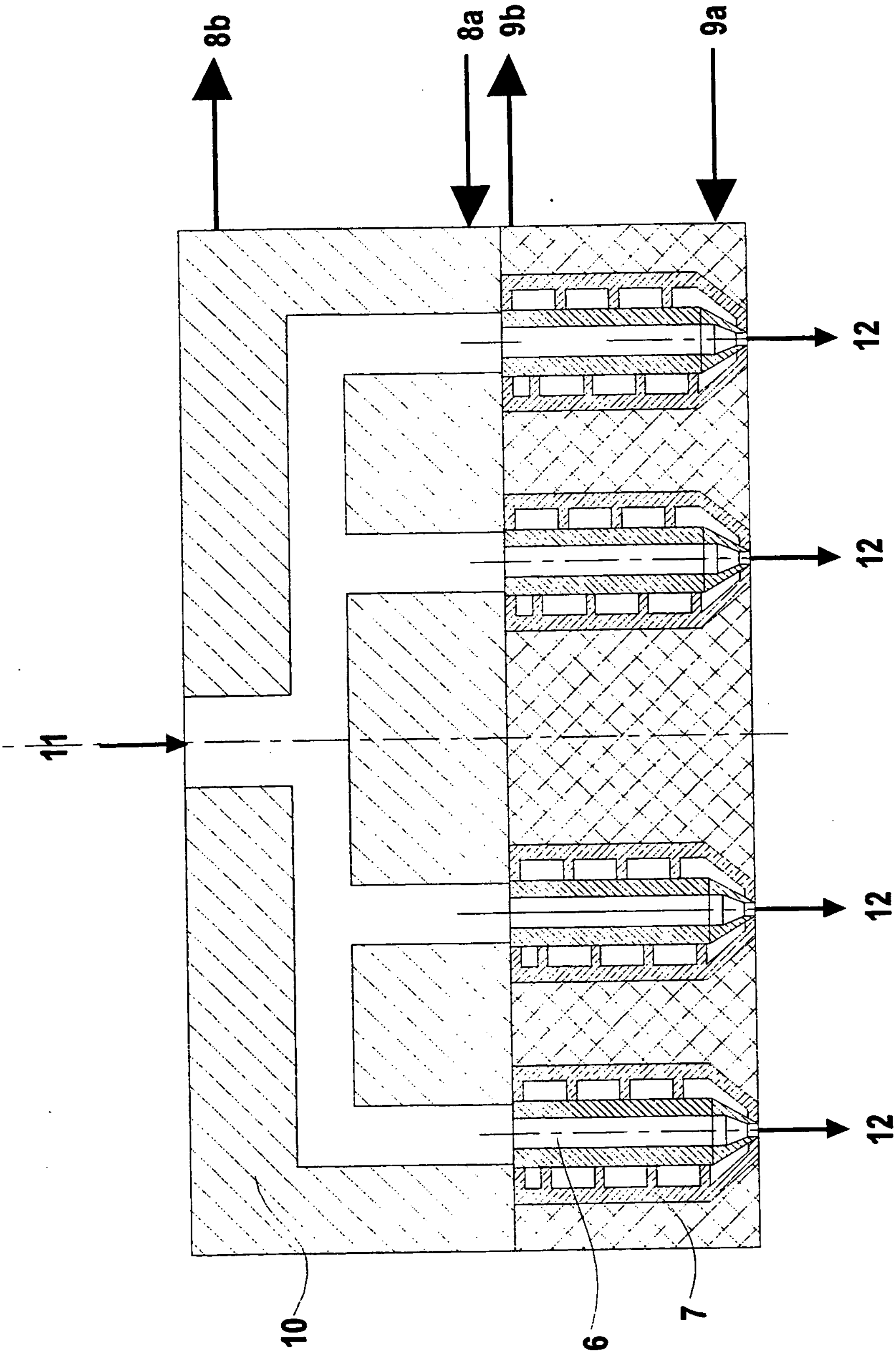


Figur 5

Figur 6

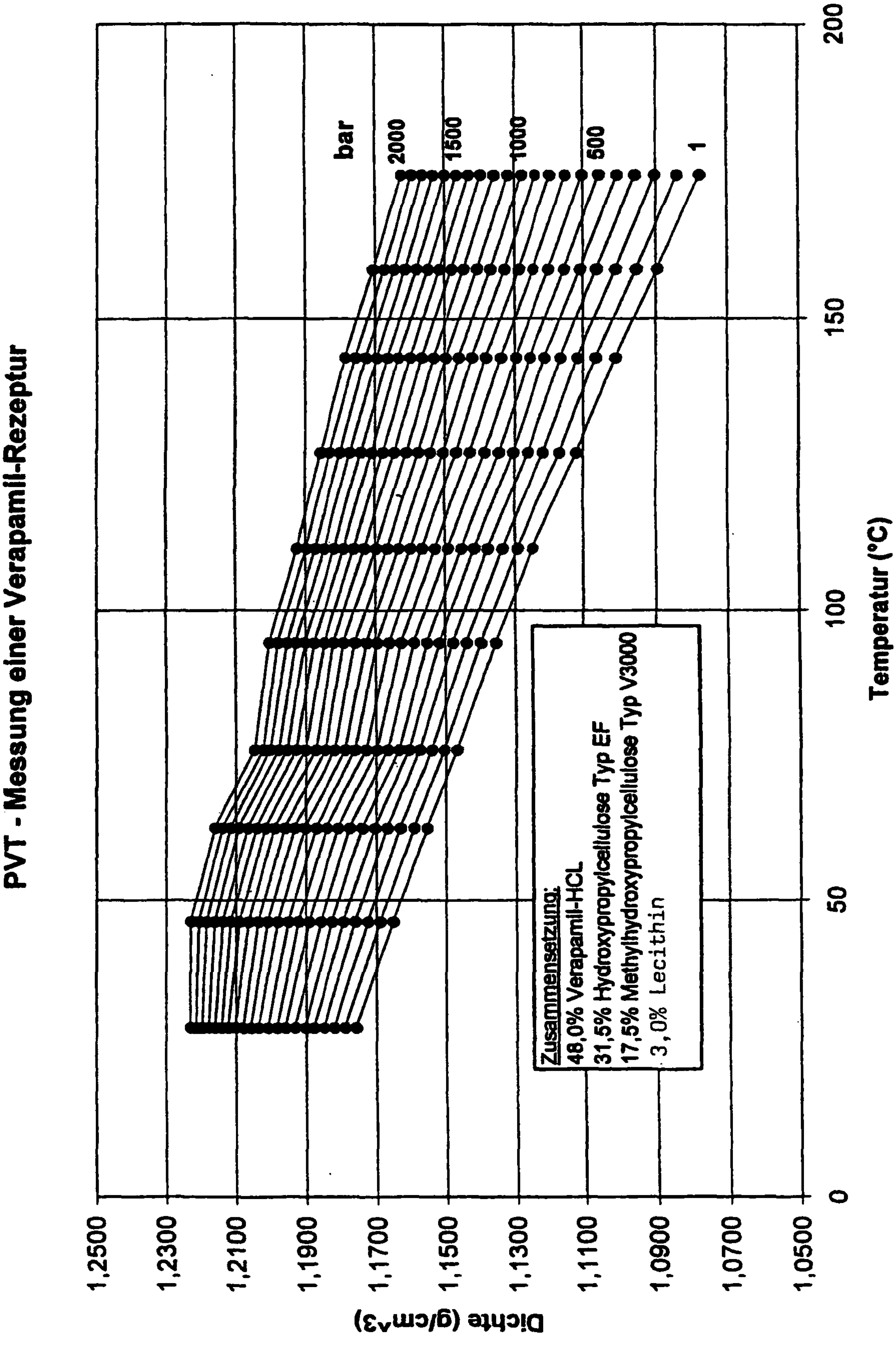
PVT - Messung einer Verapamil-Rezeptur
Dichte (g/cm^3)
1,2500
1,2300
1,2100
1,1900
1,1700
1,1500
1,1300
1,1100
1,0900
1,0700
1,0500
Temperatur (°C)
0
50
100
150
200
bar
2000
1500
1000
500
1
Zusammensetzung:
48,0% Verapamil-HCL
31,5% Hydroxypropylcellulose Typ EF
17,5% Methylhydroxypropylcellulose Typ V3000
3,0% Lecithin

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 9715268 A
- WO 9511122 A1
- WO 9841381 A1
- EP 0337256 A2
- EP 240904 B1


### In der Beschreibung aufgeführte Nicht-Patentliteratur


- **W.D. RHINE et al.** Controlled Release of Bioactive Materials. Academic Press, 1980
- **H. FIKENTSCHER.** *Cellulose-Chemie,* 1932, vol. 13, 58-6471-74
- **H. SUCKER et al.** Pharmazeutische Technologie. Thieme-Verlag, 1978
- **J. L. FORD.** *Pharm. Acta Helv.,* 1986, vol. 61, 69-88
- **K. THOMA et al.** *Pharm. Ind.,* 1989, vol. 51, 98-101